# EUROPEAN PATENT SPECIFICATION

(11) **EP 3 887 828 B1**
(45) Date of publication and mention of the grant of the patent: **31.01.2024**
(21) Application number: 19892916.8
(22) Date of filing: 02.12.2019
(51) Int. Cl.: G01N 33/558, G01N 33/72, G01N 27/327, C12Q 1/00, G01N 33/543, G01N 33/487

(54) **SYSTEMS AND METHODS FOR A COMBINED STRIP DETECTION AND HEATING SYSTEM IN AN ELECTROCHEMICAL TEST STRIP**
SYSTEME UND VERFAHREN FÜR EIN KOMBINIERTES STREIFENDETEKTIONS- UND -HEIZSYSTEM IN EINEM ELEKTROCHEMISCHEN TESTSTREIFEN
SYSTÈMES ET PROCÉDÉS POUR UN SYSTÈME COMBINÉ DE DÉTECTION ET DE CHAUFFAGE DE BÂTONNET DANS UN BÂTONNET DIAGNOSTIQUE ÉLECTROCHIMIQUE

(30) Priority: 02.12.2018 US 201862774292 P
(43) Date of publication of application: 06.10.2021
(73) Proprietor: Polymer Technology Systems, Inc., Whitestown, IN 46075 (US)
(72) Inventor: JOYCE, Joseph P., Lafayette, Indiana 47905 (US)
(74) Representative: Stöckeler, Ferdinand
(86) International application number: PCT/US2019/064062
(87) International publication number: WO 2020/117709

(56) References cited:
- WO-A1-02/50534
- US-A1- 2003 079 987
- US-A1- 2012 295 269
- US-A1- 2013 026 051
- US-A1- 2013 098 777
- LIN HUA ET AL: "Current Status of HbA1c Biosensors", SENSORS, vol. 17, no. 8, 1 January 2017 (2017-01-01), page 1798, XP55941163, DOI: 10.3390/s17081798 Retrieved from the Internet: URL:https://www.ncbi.nlm.nih.gov/pmc/artic les/PMC5579747/pdf/sensors-17-01798.pdf>

## Description

### BACKGROUND

In many scenarios, doctors, consumers, and health professionals desire to test for various analytes. Although lab testing is readily available for users, such testing requires users to send away samples and results will not usually be ready quickly. Therefore, point of care (POC) testing systems are desirable. Some of the biggest factors affecting the sale and use of POC testing systems is the convenience, disposability, and ease of use provided by various systems. Therefore, systems that provide such factors are highly desirable.

WO 02/50534 A1 and US 2003/079987 A1 disclose a sensor apparatus in which electrodes may be used to heat a sample, but do not disclose use of the apparatus in relation to Hb A1C. Lin and Yi (Sensors (Basel). 2017 Aug; 17(8): 1798) reviews Hb A1C biosensors, but does not disclose using the detecting electrode to heat the sample.

### BRIEF SUMMARY

The invention provides a system adapted for testing for an analyte according to claim 1 and a method of heating a sample in a test strip according to claim 11. Further developments of the invention are defined in the dependent claims.

### BRIEF DESCRIPTION OF THE DRAWINGS

Fig. 1 shows a diagram of an embodiment of a combined strip detection and heating system in an electrochemical test strip;
Fig. 2 shows a perspective view of a diagram of the combined strip detection and heating system in an electrochemical test strip of Fig. 1;
Fig. 3A-3B show additional diagrams of the e-gated combined strip detection and heating system in an electrochemical test strip of Fig. 1;
Fig. 4 shows another diagram of one embodiment of a combined strip detection and heating system in an electrochemical test strip;
Fig. 5 shows another embodiment of a test strip for an ICA (Integrated Consumable Assay);
Fig. 6 shows the flow areas for the test strip of Fig. 5.

### DETAILED DESCRIPTION

Certain terminology is used herein for convenience only and is not to be taken as a limitation on the embodiments of the system and methods for combined strip detection and heating system in an electrochemical test strip. Essentially, the test strip includes a test strip detection system that also is used as a heating element for a sample. In many embodiments, the system tests for A1C and Hb. In many embodiments, the A1C sample must be heated in order to digest the sample and this is done via the combined strip detection and heating system in an electrochemical test strip.

In many embodiments, an important functional step of the assay is to heat the prepared A1C sample to digest the hemoglobin, prior to contact with the electrode area.

To meet cost and ease of use targets the biosensor strip is required to be a highly integrated, self-functional component of the overall product design. Similar to how an IC chip is to a PCB Assembly. After "analysis-for-integration," it was determined that the strip detect and the fill detect conductors on the biosensor strip could be "multi-purposed" for another design requirement; heating. The chemistry requires that the A1C lysed blood sample be heated to a temperature that significantly decreases the reaction time required. Therefore, in many embodiments, the conductive gold could be set up to be the resistive heating element needed for the chemistry requirements.

In many embodiments, the biosensor strip uses the strip detect conductor and the fill detect conductor to heat the sample to required temperature. The strip conductors are now applied with a voltage at intervals of time to generate a heat output (thermal watts) in order increase the chemistry temperature. Via an e-gate, the sample is typically held in the heating area. Alternative techniques are possible for holding the sample an area, including blister packs, mechanical gates, or dissolvable barriers.

Additionally, the system may include an e-gating feature in an electrochemical test strip (also referred to as an e-gated test strip or EGTS) for keeping the sample in a heating area. In many embodiments, an e-gated test strip includes a braking feature. By braking feature, it is meant to refer to a feature that stops or slows the flow of fluid in the test strip such that it does not completely advance into another area of the test strip. In many configurations, such braking features are implemented in a test strip utilizing microfluidics and/or capillary flow tubes. In many embodiments the braking feature is a hydrophobic glass bead dielectric that can be applied (screened) on top of the standard electric contacts on-strip. The thickness, wetting angle, feature geometry, potential applied, and channel height play important roles in the effectiveness of the design. In many embodiments, the braking feature is the ablated trough that is cut into the body of the base polymer film that the biosensor is fabricated from. The trough is a semicircular feature that has an approximate 1 to 1 ratio of diameter to fluid channel width. The depth and edge "sharpness" play a role in the reliability and longevity of the fluid to be held in place until the analyzer releases the fluid to the next stage. In many embodiments, the braking feature is a Self-Assembled Monolayer (SAM). This is a printed (screened or flood) process application that needs a temporary holding dam so that the fluid carrier can allow the features of the SAM to assemble properly and then remove the dam for the next component to be assembled. In many embodiments, the braking features do not need any external forces to function and stop the flow of fluid. Instead they function automatically based on current applied via electrodes from a meter. In alternatives, other braking techniques may be used for the e-gate that may be actuated by a current and do not require mechanical pieces.

In many embodiments, the combined strip detection and heating system test strip is used in the detection of hemoglobin and hemoglobin A1C such that a ratio of the two is calculated. As part of this assay, in many configurations, the flow of the lysed sample is controlled in order to provide a proper sequence in the system. In many configurations, the combined strip detection and heating system test strip is constructed as a multi-layer film strip biosensor. In many configurations, the biosensor has highly engineered film layers that control biosensor detection, electrical signals, fluid flow and heat. Important to this application is the control of fluid flow and location.

In some embodiment, the combined strip detection and heating system test strip integrates one of three differently fabricated, in-process braking features to provide a system that gives greatest reliability for control, near and long term. Of them, two of the three designs utilize an applied low voltage to the leading edge of the fluid to alter the wetting angle of the fluid so that the braking feature geometry is now misaligned and the capillary action of the wetted surfaces pulls or restarts the flow of the fluid into the measurement channel. The third braking feature takes advantage of the hydrophobic nature of a Self-Assembled Monolayer (SAM). The SAM has the ability to relax its hydrophobic characteristics due to an applied voltage and allow the fluid to restart into the measurement channel.

In many embodiments, the first braking feature is a hydrophobic glass bead dielectric that can be applied (screened) on top of the standard electric contacts on-strip. The thickness, wetting angle, feature geometry, potential applied, and channel height play important roles in the effectiveness of the design.

In many embodiments, the second braking feature design is the ablated trough that is cut into the body of the base polymer film that the biosensor is fabricated from. The trough is a semicircular feature that has an approximate 1 to 1 ratio of diameter to fluid channel width. The depth and edge 'sharpness" play a role in the reliability and longevity of the fluid to be held in place until the analyzer releases the fluid to the next stage.

In many embodiments, the third braking feature is the Self-Assembled Monolayer (SAM). This is a printed (screened or flood) process application that needs a temporary holding dam so that the fluid carrier can allow the features of the SAM to assemble properly and then remove the dam for the next component to be assembled.

Numerous advantages exist for the combined strip detection and heating system, including:
1.) Increased consumer use reliability; less components lead to less process failure
2.) Lower inventory requirements and manufacturing time.
3.) Lower product cost
4.) Lower shipping packaging and transit costs.
5.) The on-strip heating allows for reduced physical component tolerance stack up.
6.) It applies heat directly to the sample, improving consistency.

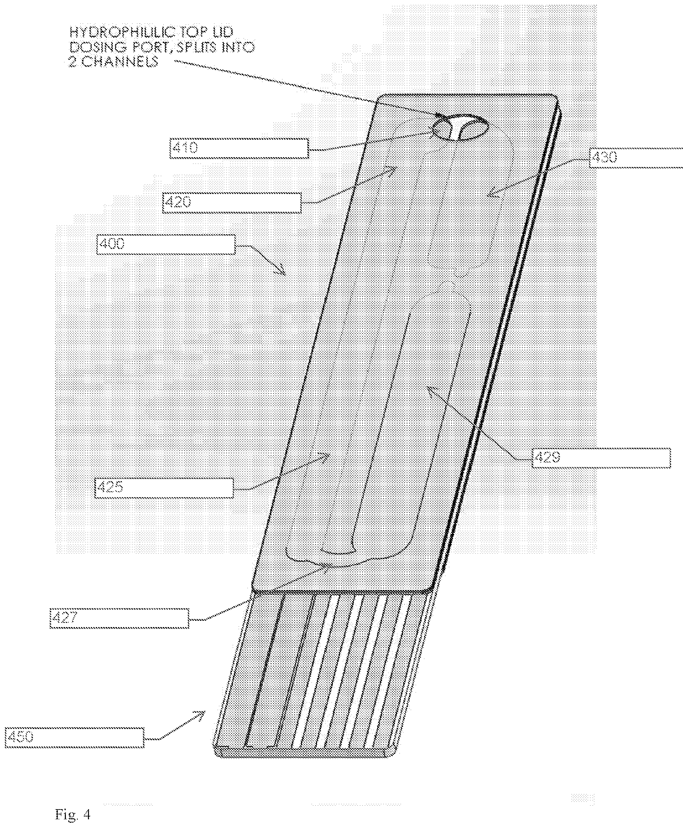

Fig. 4 shows a diagram of one embodiment of the combined strip detection and heating system test strip 400. In this diagram, the application port 410 is visible. Application port 410 is located in a hydrophobic cover for the strip. Also visible are the two flow channels of the device, A1C channel 420 and Hb channel 430. In A1C channel 420 there two primary areas, heating area 425 and detection area 429 which are separated by an e-gate 427. In the heating area, the sample is heated via the combined strip detection and heating system. Also, the electrode leads 450 are visible. This setup is used because the Hb channel does not require heating/digestion, so the sample can proceed immediately to the detection area, whereas in the A1C channel 420, digestion is first necessary. The trace of the various circuitry is not visible in this configuration.

In many embodiments, in heating area 425, the sample is heated and digested. This may occur according to a variety of techniques; however, it is important to prevent the advance of the sample beyond. This is the purpose of the e-gate.

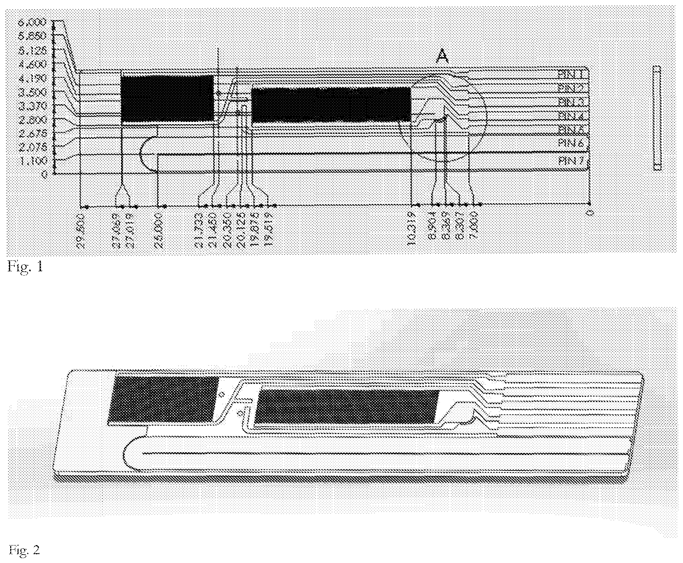

Table 1 below shows an explanation of the process flow for embodiments of the combined strip detection and heating system test strip. Typically, circuitry/logic in a meter controls the operation of the test strip, however, such control circuity/logic may be resident in any device that engages the test strip. As shown in the table below and Fig. 1 and 2, the electrode and pin arrangement are shown. When the test strip is first introduced into the meter and the meter is activated, the system detects the strip via pin 6 and 7. This is event 1. These pins, 6 and 7, are part of a u-shaped trace. Pin 5 and pin 6 detect that heating area 425 has been filled. This is event 2. Pin 1 and pin 2 are provided for use in measuring the hemoglobin in the sample provided. This is event 3. Subsequently, in event 4, pin 6 and pin 7 cooperate to heat the sample for the A1C side of the test in event 4. Once the sample is sufficiently heated, the pins 4, 5, and 6 are used to release the e-gate, in event 5. In event 6, Pin 2 and 5 are used to detect a fill of the A1C detection area 429. Subsequently, in event 7, pin 3 and pin 4 provide for the detection and measurement of A1C, providing a counter electrode and a working electrode.

| PIN # | CHEMISTRY | Signal | | | | | | |
|---|---|---|---|---|---|---|---|---|
| | | Event 1 | Event 2 | Event 3 | Event 4 | Event 5 | Event 6 | Event 7 |
| 1 | Hb | | | Counter | | | | |
| 2 | Hb | | | Working | | | Fill A1C | |
| 3 | A1C | | | | | | | Counter |
| 4 | A1C | | | | | Release E-Gate | | Working |
| 5 | A1C | | Heat Channel Fill | | | Release E-Gate | Fill A1C | |
| 6 | A1C | Strip Detect | Heat Channel Fill | | Heat | Release E-Gate | | |
| 7 | | Strip Detect | | | Heat | | | |

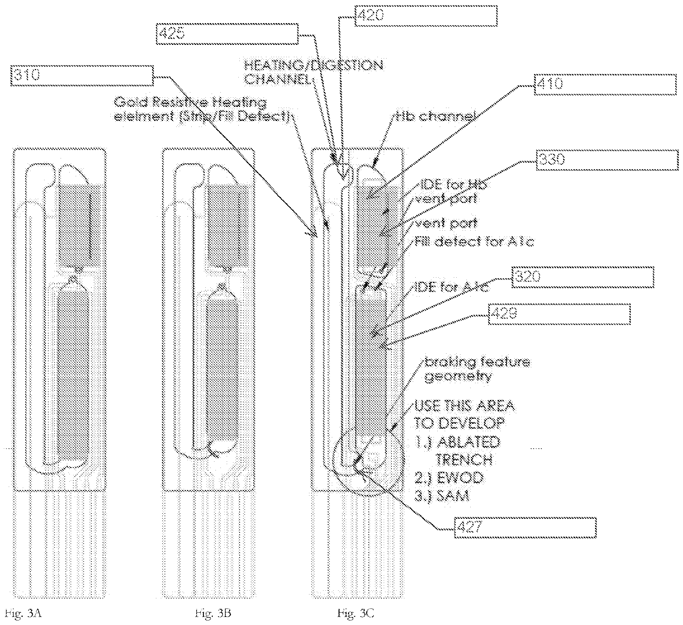

Fig. 3A-3C provide more detail concerning the system. As shown in 3C, a heating/digestion channel or heating area 425 425on the A1C channel 420 side is provided. Under this channel is the gold resistive heating element 310 that also provides for the detection of the insertion of the strip. This is one possible embodiment of the combined strip detection and heating system. Other resistive elements are possible, that will provide heat and detection of insertion. One of three possible techniques for e-gating is used at e-gate 427. Subsequently, in detection area 429 an interdigitated electrode 320 for A1C is located that allows for the measurement of A1C. On the Hb channel 320, a Hb interdigitated electrode 330 is provided for the measurement of hemoglobin.

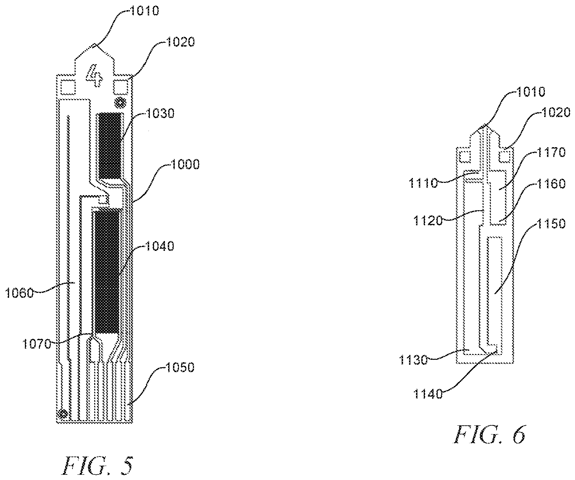

Fig. 5 shows another embodiment of a test strip for an ICA, specifically the substrate piece. This test strip 1000 is specifically designed to include a piercing end 1010. The strip also includes a stop 1020 to prevent the strip from advancing too far. In this embodiment, the piercing end 1010 is designed to pierce a reservoir that holds reactants or lysing agents (or some other material for interacting with the sample). Additionally, in this view, electrode areas 1030, 1040 are shown. In many embodiments these are interdigitated electrodes but other configurations are possible. Leads 1050 interact with a meter in order to power the strip to detect the analyte of interest. In alternatives, the strip may still include the piercing end 1010, however may be an optical strip. Electrodes 1060 provides for a hearing element, that may heat a sample by powering the electrodes. Additionally, at finger 1140 shown in Fig. 6, the two leads located at 1070 provide electrical energy to electrically active the e-gate located at this area.

Fig. 6 shows the flow path etching that is laid over the substrate piece of Fig. 6. Additionally, a cover, not show, is laid over the substrate. Similarly, the device includes pierce point 1010 that aligns with the pierce point 1010 on the substrate. The strip also includes a stop 1020 to prevent the strip from advancing too far. Typically the channels of the device rely on capillary type flow action and include vents 1130, 1160 in the cover that is laid over the spacer. The device includes a digestion finger 1110 and digestion channel 1120, where the sample may be heated such that digestion can occur. In alternative embodiments, other chemical procedures may occur in this channel. A1C finger 1140 leads to electrode area 1040 where electrochemical testing may occur. The A1C channel 1150 lead to the area over electrode area 1040. Additionally, including is a channel that does not require digestion, channel 1170 over electrode 1030. This channel 1170 includes a vent 1160 in the cover. In many embodiments this channel is a hemoglobin channel.

Therefore, in many embodiments, a test strip is provided that has a single stage electrochemical detection system and a dual stage electrochemical detection system. A single port leads to both electrochemical detection systems and the flow paths for each. On the single stage electrochemical detection system side, the flow path leads to a working electrode and counter electrode arrangement, whereby a level of a first analyte may be detected. One the dual stage electrochemical detection system side, the flow path first leads to a heating/digestion area. This area is heated by the combined strip detection and heating system. This area is separated from a detection area via an e-gate. The e-gate may automatically be opened and triggered by the meter (or other electrical source) connected to the test strip. Once the sample is heated/digested sufficiently, the gate is opened and the sample proceeds to the detection area. In many embodiments the single stage electrochemical detection system is for Hb. In many embodiments, the dual stage electrochemical detection system is for A1C.

In many embodiments, a meter associated with the combined strip detection and heating system test strip includes logic for running the test, including, but not limited to, logic for detecting when/whether the strip is inserted (performed using the combined strip detection and heating system); logic for beginning the testing; logic for determining when the heating area is filled; logic for activating the heating element in the heating area (using the combined strip detection and heating system); logic for releasing the e-gate when the sample is sufficiently digested/heated; logic for determining when the A1C testing area is full; logic for activating the electrodes for determining A1C levels; logic for determining when the Hb testing area is full; ; logic for activating the electrodes for determining A1C levels; logic for outputting a result (in many scenarios including the ratio of Hb/A1C). In many scenarios, this logic includes activating and deactivating various electrodes/leads by providing current or voltage to the pins as discussed above.

In many embodiments, parts of the system are provided in devices including microprocessors. Various embodiments of the systems and methods described herein may be implemented fully or partially in software and/or firmware. This software and/or firmware may take the form of instructions contained in or on a non-transitory computer-readable storage medium. Those instructions then may be read and executed by one or more processors to enable performance of the operations described herein. The instructions may be in any suitable form such as, but not limited to, source code, compiled code, interpreted code, executable code, static code, dynamic code, and the like. Such a computer-readable medium may include any tangible non-transitory medium for storing information in a form readable by one or more computers such as, but not limited to, read only memory (ROM); random access memory (RAM); magnetic disk storage media; optical storage media; a flash memory, etc.

Embodiments of the systems and methods described herein may be implemented in a variety of systems including, but not limited to, smartphones, tablets, laptops, and combinations of computing devices and cloud computing resources. For instance, portions of the operations may occur in one device, and other operations may occur at a remote location, such as a remote server or servers. For instance, the collection of the data may occur at a smartphone, and the data analysis may occur at a server or in a cloud computing resource. Any single computing device or combination of computing devices may execute the methods described.

## Claims

1. A system adapted for testing for an analyte, the analyte being Hb A1C, the system comprising:
a test strip including:
a test strip detection conductor;
a first flow path, the first flow path including a heating area, the test strip detection conductor in the heating area, the test strip detection conductor configured to be activated to heat a sample in the heating area.

2. The system of claim 1, wherein the test strip detection conductor is a gold resistive heating element.

3. The system of claim 2, wherein test strip further includes an e-gate, the e-gate in the first flow path, the e-gate separating the heating area from a detection area of the first flow path, the e-gate comprising a braking feature that stops or slows the flow of fluid in the test strip such that it does not completely advance into another area of the test strip.

4. The system of claim 3, wherein the test strip further includes a second flow path, the second flow path including an interdigitated electrode for detecting hemoglobin.

5. The system of claim 4, further comprising a meter, which engages the test strip and is configured to detect the test strip via the test strip detection conductor.

6. The system of claim 5, wherein the meter is configured to provide current to the test strip detection conductor and heat the sample in the heating area.

7. The system of claim 6, wherein the meter is configured to open the e-gate when the sample has reached a necessary temperature.

8. The system of claim 6, wherein the meter is configured to open the e-gate after the sample has undergone necessary digestion.

9. The system of claim 6, wherein the meter is configured to provide current to the test strip detection conductor until the sample has reached a necessary temperature.

10. The system of claim 6, wherein the meter is configured to provide current to the test strip detection conductor until the sample has undergone necessary digestion.

11. A method of heating a sample in a test strip adapted for testing for HbAlC, the method comprising:
providing a test strip;
applying a sample to the test strip;
slowing the flow of the sample in a heating area of a first flow path; and
activating a test strip detection conductor in the heating area to heat the sample.

12. The method of claim 11, further comprising:
detecting insertion of the test strip into a meter using the test strip detection conductor.

13. The method of claim 12, wherein the detecting including the meter providing a current to the test strip detection conductor.

## Patentansprüche

1. Ein System, das zum Testen auf einen Analyten angepasst ist, wobei der Analyt HbA1c ist, wobei das System folgende Merkmale aufweist:
einen Teststreifen, der folgende Merkmale aufweist:
einen Teststreifendetektionsleiter;
einen ersten Strömungspfad, wobei der erste Strömungspfad einen Wärmebereich umfasst, wobei sich der Teststreifendetektionsleiter in dem Erwärmungsbereich befindet, wobei der Teststreifendetektionsleiter dazu konfiguriert ist, aktiviert zu werden, um eine Probe in dem Erwärmungsbereich zu erwärmen.

2. Das System gemäß Anspruch 1, wobei der Teststreifendetektionsleiter ein Goldwiderstandserwärmungselement ist.

3. Das System gemäß Anspruch 2, wobei der Teststreifen ferner ein e-Gate umfasst, wobei das e-Gate in dem ersten Strömungspfad ist, wobei das e-Gate den Erwärmungsbereich von einem Detektionsbereich des ersten Strömungspfades trennt, wobei das e-Gate ein Bremsmerkmal aufweist, das die Strömung eines Fluids in dem Teststreifen derart stoppt oder verlangsamt, dass dasselbe nicht vollständig in einen anderen Bereich des Teststreifens fortschreitet.

4. Das System gemäß Anspruch 3, wobei der Teststreifen ferner einen zweiten Strömungspfad umfasst, wobei der zweite Strömungspfad eine verschränkte Elektrode zum Detektieren von Hämoglobin umfasst.

5. Das System gemäß Anspruch 4, das ferner ein Messgerät aufweist, welches den Teststreifen in Eingriff nimmt und dazu konfiguriert ist, den Teststreifen über den Teststreifendetektionsleiter zu detektieren.

6. Das System gemäß Anspruch 5, wobei das Messgerät dazu konfiguriert ist, dem Teststreifentestleiter Strom zuzuführen und die Probe in dem Erwärmungsbereich zu erwärmen.

7. Das System gemäß Anspruch 6, wobei das Messgerät dazu konfiguriert ist, das e-Gate zu öffnen, wenn die Probe eine notwendige Temperatur erreicht hat.

8. Das System gemäß Anspruch 6, wobei das Messgerät dazu konfiguriert ist, das e-Gate zu öffnen, nachdem die Probe einer notwendigen Digestion unterzogen wurde.

9. Das System gemäß Anspruch 6, wobei das Messgerät dazu konfiguriert ist, dem Teststreifendetektionsleiter Strom zuzuführen, bis die Probe eine notwendige Temperatur erreicht hat.

10. Das System gemäß Anspruch 6, wobei das Messgerät dazu konfiguriert ist, dem Teststreifendetektionsleiter Strom zuzuführen, bis die Probe einer notwendigen Digestion unterzogen wurde.

11. Ein Verfahren zum Erwärmen einer Probe in einem Teststreifen, der angepasst ist zum Testen auf HbA1C, wobei das Verfahren folgende Schritte aufweist:
Bereitstellen eines Teststreifens;
Auftragen einer Probe auf den Teststreifen;
Verlangsamen der Strömung der Probe in einem Erwärmungsbereich eines ersten Strömungspfades; und
Aktivieren eines Teststreifendetektionsleiters in dem Erwärmungsbereich, um die Probe zu erwärmen.

12. Das Verfahren gemäß Anspruch 11, das ferner folgenden Schritt aufweist:
Detektieren einer Einführung des Teststreifens in ein Messgerät unter Verwendung des Teststreifendetektionsleiters.

13. Das Verfahren gemäß Anspruch 12, wobei das Detektieren umfasst, dass das Messgerät dem Teststreifendetektionsleiter Strom zuführt.

## Revendications

1. Système adapté pour tester un analyte, l'analyte étant Hb A1C, le système comprenant:
une bandelette de test comportant:
un conducteur de détection de bandelette de test;
un premier trajet de circulation, le premier trajet de circulation comportant une zone de chauffage, le conducteur de détection de bandelette de test se trouvant dans la zone de chauffage, le conducteur de détection de bandelette de test étant configuré pour être activé pour chauffer un échantillon dans la zone de chauffage.

2. Système selon la revendication 1, dans lequel le conducteur de détection de bandelette de test est un élément chauffant résistant à l'or.

3. Système selon la revendication 2, dans lequel la bandelette de test comporte par ailleurs une porte électronique, la porte électronique se trouvant dans le premier trajet de circulation, la porte électronique séparant la zone de chauffage d'une zone de détection du premier trajet de circulation, la porte électronique comprenant une caractéristique de freinage qui arrête ou ralentit la circulation du fluide dans la bandelette de test de sorte qu'il n'avance pas complètement dans une autre zone de la bandelette de test.

4. Système selon la revendication 3, dans lequel la bandelette de test comporte par ailleurs un deuxième trajet de circulation, le deuxième trajet de circulation comportant une électrode interdigitée destinée à détecter de l'hémoglobine.

5. Système selon la revendication 4, comprenant par ailleurs un compteur qui vient en prise avec la bandelette de test et est configuré pour détecter la bandelette de test par l'intermédiaire du conducteur de détection de bandelette de test.

6. Système selon la revendication 5, dans lequel le compteur est configuré pour fournir du courant au conducteur de détection de bandelette de test et pour chauffer l'échantillon dans la zone de chauffage.

7. Système selon la revendication 6, dans lequel le compteur est configuré pour ouvrir la porte électronique lorsque l'échantillon a atteint une température nécessaire.

8. Système selon la revendication 6, dans lequel le compteur est configuré pour ouvrir la porte électronique après que l'échantillon a subi la digestion nécessaire.

9. Système selon la revendication 6, dans lequel le compteur est configuré pour fournir du courant au conducteur de détection de bandelette de test jusqu'à ce que l'échantillon ait atteint une température nécessaire.

10. Système selon la revendication 6, dans lequel le compteur est configuré pour fournir du courant au conducteur de détection de bandelette de test jusqu'à ce que l'échantillon ait subi la digestion nécessaire.

11. Procédé de chauffage d'un échantillon dans une bandelette de test adaptée pour tester Hb A1C, le procédé comprenant le fait de:
prévoir une bandelette de test;
appliquer un échantillon sur la bandelette de test;
ralentir la circulation de l'échantillon dans une zone de chauffage d'un premier trajet de circulation; et
activer un conducteur de détection de bandelette de test dans la zone de chauffage pour chauffer l'échantillon.

12. Procédé selon la revendication 11, comprenant par ailleurs le fait de:
détecter l'insertion de la bandelette de test dans un compteur à l'aide du conducteur de détection de bandelette de test.

13. Procédé selon la revendication 12, dans lequel la détection comporte le fait que le compteur fournit un courant au conducteur de détection de bandelette de test.
